(19) Europäisches Patentamt
European Patent Office
Office européen des brevets

(11) Veröffentlichungsnummer : **0 149 781**
**B1**

(12) # EUROPÄISCHE PATENTSCHRIFT

(45) Veröffentlichungstag der Patentschrift : 22.02.89

(21) Anmeldenummer : 84115042.8

(22) Anmeldetag : 10.12.84

(51) Int. Cl.⁴ : **C 07 D405/12**, C 07 D405/14, C 08 K 5/34

ERRATUM

| DIE TEXTSTELLE : TEXT PUBLISHED : LE PASSAGE SUIVANT : | (SEITE, SPALTE, ZEILE) (PAGE, COLUMN, LINE) (PAGE, COLONNE, LIGNE) | | | LAUTET BERICHTIGT: SHOULD READ : DEVRAIT ETRE LU : |
|---|---|---|---|---|
| Die EP-PS 20 40 975 | 2 | | 7 | Die DE-PS 20 40 975 |
| $R^7$ bis $R^{10}$ $C_1$- bis $C_4$-Alkyl, | 3 | | 2 | $R^7$ bis $R^{10}$ $C_1$ bis $C_4$-Alkyl, oder |
| $R^7$ und $R^8$ oder $R^9$ und $R^{10}$ | 3 | | 3 | $R^7$ und $R^8$ und $R^9$ und $R^{10}$ |
| $(CH_2)_2O(CH_2)_2OC_2CH_5$, | 3 | | 20 | $(CH_2)_2O(CH_2)_2OC_2H_5$, |
| $(C_2H_4O_3)C_2H_5$, | 3 | | 21 | $(C_2H_4O)_3C_2H_5$, |
| wird durch Ausrühen | 6 | | 34 | wird durch Ausrühren |
| im Wasserstrahlvolumen | 6 | | 58 | im Wasserstrahlvakuum |

Tag der Entscheidung über die Berichtigung / Date of decision on rectification: / Date de décision portant sur modification: 24.05.89

Ausgabe- und Veröffentlichungstag: / Issue and publication date: / Date d'edition et de publication: 23.08.89

Patbl.Nr) / EPB no:) 89/34 / Bull. no:)

(19) Europäisches Patentamt
European Patent Office
Office européen des brevets

(11) Veröffentlichungsnummer : **0 149 781 B1**

(12) # EUROPÄISCHE PATENTSCHRIFT

(45) Veröffentlichungstag der Patentschrift : **22.02.89**

(51) Int. Cl.[4] : **C 07 D405/12**, C 07 D405/14, C 08 K 5/34

(21) Anmeldenummer : **84115042.8**

(22) Anmeldetag : **10.12.84**

(54) **Furan-3-carbonsäurederivate.**

(30) Priorität : **15.12.83 DE 3345376**

(43) Veröffentlichungstag der Anmeldung : **31.07.85 Patentblatt 85/31**

(45) Bekanntmachung des Hinweises auf die Patenterteilung : **22.02.89 Patentblatt 89/08**

(84) Benannte Vertragsstaaten : **CH DE FR GB IT LI NL**

(56) Entgegenhaltungen :
**WO-A-81 /020 21**
**DE-A- 2 258 752**
**DE-B- 2 040 975**
**DE-B- 2 349 962**

(73) Patentinhaber : **BASF Aktiengesellschaft**
**Carl-Bosch-Strasse 38**
**D-6700 Ludwigshafen (DE)**

(72) Erfinder : **Neumann, Peter, Dr. Chemiker**
**Franz-Schubert-Strasse 1**
**D-6908 Wiesloch (DE)**
Erfinder : **Helwig, Reinhard, Dr. Chemiker**
**Bruesseler Ring 4**
**D-6700 Ludwigshafen (DE)**
Erfinder : **Weiss, Stefan, Dr. Chemiker**
**Carl-Beck-Strasse 46**
**D-6903 Neckargemuend (DE)**
Erfinder : **Trauth, Hubert, Diplom-Ingenieur**
**Milanstrasse 5**
**D-6724 Dudenhofen (DE)**

**EP 0 149 781 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Es ist bereits bekannt, daß 2,2,6,6-Tetraalkylpiperidinderivate ausgezeichnete Lichtschutzmittel für organische Polymere sind. Unter ihnen sind auch Furancarbonsäurederivate bekannt geworden.

In der DE-OS 22 58 752 sind Tetraalkylpiperidinylester der Furan-2-carbonsäure und der Furan-2,5-dicarbonsäure beschrieben. Die Veröffentlichung WO 81 02 021 erwähnt den Tri-(2,2,6,6-tetramethyl-4-piperidinyl)-ester einer nicht genau definierten Furantricarbonsäure. Die Offenlegungsschrift DE-OS 26 23 422 schließlich beschreibt Tetraalkylpiperidinylester der Furan-2-carbonsäure.

Die EP-PS 20 40 975 beansprucht Tetraalkylpiperidinylamide der Furan-2-carbonsäure und der Furan-2,3,4-tricarbonsäure, während die DE-PS 23 49 962 Pentaalkylpiperidinylamide der Furan-2-carbonsäure beschreibt.

Gemeinsames Merkmal der bisher beschriebenen Furancarbonsäure-2,2,6,6-tetraalkyl-4-piperidinylester ist wenigstens eine Carboxylgruppe in der 2-Stellung des heterocyclischen Rings. Überraschenderweise wurde gefunden, daß die erfindungsgemäßen 2,2,6,6-Tetraalkyl-4-piperidinylderivate alkylsubstituierter Furan-3-carbonsäuren in ihrer stabilisierenden Wirkung den entsprechenden Furan-2-carbonsäurederivaten deutlich überlegen sind.

Die Erfindung betrifft nun Furancarbonsäurederivate der allgemeinen Formel

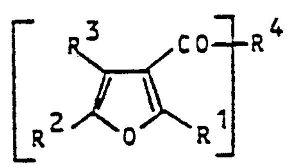

in der

$R^1$ bis $R^3$ unabhängig voneinander Wasserstoff, $C_1$-bis $C_8$-Alkyl, Cyclohexyl oder Phenyl, jedoch nicht mehr als zwei dieser Reste gleichzeitig Wasserstoff und

$R^4$ ein Rest der Formel

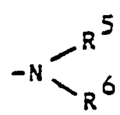 , 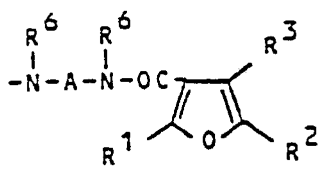

oder $OR^6$ sind, wobei

A lineares oder verzweigtes, gegebenenfalls ein oder mehrere Ethersauerstoff enthaltendes $C_2$- bis $C_{12}$-Alkylen,

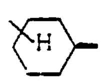 , 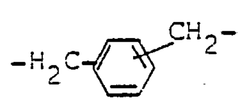 ,

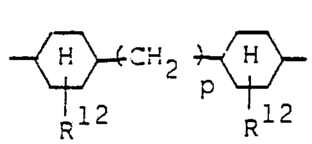 oder 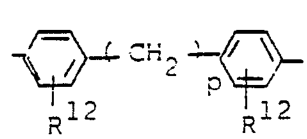

worin $R^{12}$ Wasserstoff oder Methyl und p 0, 1 oder 2 bedeuten,

$R^5$ Wasserstoff, $C_2$- bis $C_6$-Alkenyl, gegebenenfalls bis zu 3mal durch Sauerstoff unterbrochenes $C_1$- bis $C_{12}$-Alkyl oder $C_5$- bis $C_7$-Cycloalkyl und

$R^6$ ein Rest der Formel

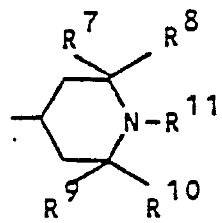

sind, in der

$R^7$ bis $R^{10}$ $C_1$-bis $C_4$-Alkyl,

$R^7$ und $R^8$ oder $R^9$ und $R^{10}$ zusammen eine Tetra- oder Pentamethylenbrücke und

$R^{11}$ Wasserstoff, $C_1$- bis $C_8$-Alkyl, $C_3$- bis $C_8$-Alkenyl, $C_2$- bis $C_4$-Hydroxyalkyl oder Aralkyl bedeuten sowie deren Salze.

Alkylreste für $R^1$ bis $R^3$, $R^5$ sowie $R^7$ bis $R^{10}$ und $R^{11}$ sind im Rahmen der allgemeinen Definitionen die geradkettigen oder verzweigten Gruppen $C_nH_{2n+1}$. Bevorzugt sind die unverzweigten Reste. Im einzelnen seien beispielsweise genannt :

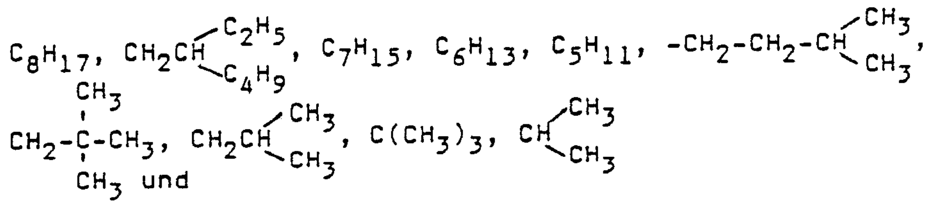

vorzugsweise $CH_3$, $C_2H_5$, $C_3H_7$ oder $C_4H_9$.

Ethersauerstoff enthaltende Alkylgruppen $R^5$ sind beispielsweise $(CH_2)_3OCH_3$, $(CH_2)_3OC_2H_5$, $(CH_2)_3OC_3H_7$, $(CH_2)_3OC_4H_9$, $(CH_2)_2O(CH_2)_2OCH_3$, $(CH_2)_2O(CH_2)_2OC_2CH_5$, $(CH_2)_2O(CH_2)_2OC_3H_7$, $(CH_2)_3O(CH_2)_2OC_4H_9$, $(C_2H_4O)_3CH_3$, $(C_2H_4O_3)C_2H_5$, $(C_2H_4O)_3C_4H_9$,

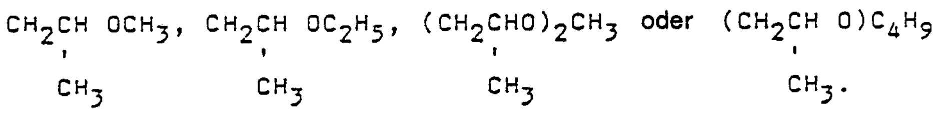

Als Alkenylgruppen sind z. B. Vinyl, Allyl, Methallyl oder 2-Butenyl zu nennen.

Cycloalkylreste für $R^5$ sind z. B. Cyclopentyl, -hexyl oder -heptyl.

Alkenyl-, Hydroxyalkyl- und Aralkylreste für $R^{11}$ sind beispielsweise $CH_2CH=CH_2$,

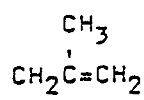

$(CH_2)_2CH=CH_2$, $(CH_2)_3CH=CH_2$, $CH_2CH_2OH$, $CH_2CHOHCH_3$, $CH_2CH_2CH_2OH$, $CH_2CHOHCH_2OH$,

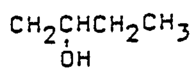

$CH_2C_6H_5$, $C_2H_4C_6H_5$, $CH_2C_6H_4CH_3$ oder $CH_2C_6H_4Cl$.

Brückenglieder A sind lineare oder verzweigte, gegebenenfalls ein oder mehrere Ethersauerstoff enthaltende $C_2$- bis $C_{12}$-Alkylengruppen sowie Reste der Formeln

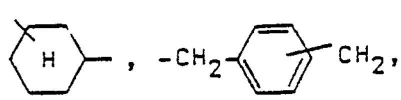

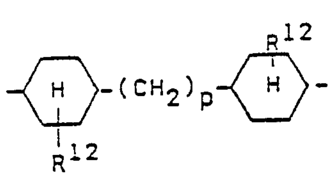

oder

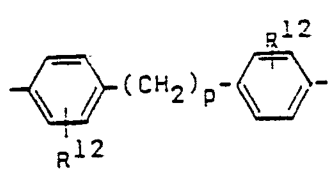

wobei $R^{12}$ Wasserstoff oder Methyl und p 0,1 oder 2 sind.

Einzelne Alkylenreste sind z. B. $C_2H_4$, $(CH_2)_3$, $(CH_2)_4$, $(CH_2)_6$, $(CH_2)_2O(CH_2)_2$, $(CH_2)_3O(CH_2)_3$ oder $(CH_2)_3O(CH_2)_2O(CH_2)_3$.

Bevorzugte Verbindungen der Formel I haben als $R^1$ bis $R^3$ Wasserstoff oder Methyl und als $R^7$ bis $R^{10}$ Methyl.

Besonders bevorzugt sind die von der 2,4- oder 2,5-Di- oder 2,4,5-Trimethylfuran-3-carbonsäure abgeleiteten Derivate.

Besonders bevorzugt sind weiterhin Verbindungen, in denen $R^1 = R^2 = CH_3$ und $R^3 = H$ und $R^7$ bis $R^{10} = CH_3$ sind.

Die erfindungsgemäßen Verbindungen können nach allgemein bekannten chemischen Verfahren hergestellt werden. Die Furancarbonsäurepiperidinylester lassen sich z. B. entweder aus den entsprechenden Säurehalogeniden und den 4-Hydroxy-2,2,6,6-tetraalkylpiperidinen in Gegenwart säurebindender Mittel, wie organischen Aminen oder Alkalihydroxiden oder aber aus den entsprechenden Methyl- oder Ethylestern durch Umesterung mit 4-Hydroxy-2,2,6,6-tetraalkylpiperidinen in Gegenwart katalytisch wirkender Verbindungen wie Alkalialkoholaten oder Titantetrabutylat herstellen. Die Herstellung der Amide erfolgt zweckmäßigerweise über die Säurehalogenide.

Die Herstellung der als Ausgangsverbindungen benötigten Furan-3-carbonsäurederivate ist literaturbekannt. So kann beispielsweise der 2,5-Dimethylfuran-3-carbonsäuremethylester nach den in den DE-OSen 22 07 098 und 28 26 013 beschriebenen Verfahren hergestellt werden.

Der 2-Methyl-5-ethylfuran-3-carbonsäureethylester und entsprechende Ester mit größeren Alkylgruppen in 5-Stellung des Furanrings sind in J. Org. Chem. 43, 4596 (1978) beschrieben.

Der 2-Methyl-5-phenylfuran-3-carbonsäureethylester kann nach der Vorschrift in Bull. Soc. Chim. France 1970, [6], 2272, hergestellt werden.

Der 2,4-Dimethylfuran- und der 2,4,5-Trimethylfuran-3-carbonsäuremethyl-ester können z. B. nach der Vorschrift in « Anales real soc. espan. fis. y quim. (Madrid) 50 B, 407-12 (1954) » [C.A. 49, 13 207 c (1955)] dargestellt werden.

Der 2-Methyl-4-ethylfuran-3-carbonsäuremethylester kann analog dem 2,4-Dimethylfuran-3-carbonsäuremethylester hergestellt werden, indem man 1-Hydroxybutan-2-on anstelle von Hydroxyaceton verwendet.

Die Synthese des 2-Methylfuran-3-carbonsäuremethylesters ist beispielsweise in der DE-OS 28 00 505 oder in J. Chem. Soc., Perkin Trans. I, 1981, 1982-9 beschrieben.

Die Herstellung weiterer alkylsubstituierter Furan-3-carbonsäureester kann der Literatur entnommen werden.

Die Herstellung der Säurechloride kann nach den bekannten Methoden erfolgen, z. B. durch Verseifung der Methyl- oder Ethylester der entsprechenden Säuren zu den freien Säuren und deren anschließender Umsetzung mit Thionylchlorid oder einer anderen geeigneten Verbindung zu den Säurechloriden.

Die erfindungsgemäßen Verbindungen können in Form der freien Basen oder aber auch als Salze vorliegen. Geeignete Anionen stammen z. B. von anorganischen Säuren und insbesondere organischen Carbonsäuren.

Anorganische Anionen sind z. B. Chlorid, Bromid, Sulfat, Methosulfat, Phosphat oder Rhodanid. Carbonsäure-Anionen sind beispielsweise : Formiat, Acetat, Propionat, Hexanoat, Cyclohexanoat, Lactat, Stearat, Dodecylbenzoat, Benzoat, Acrylat, Methacrylat, Zitrat, Malonat oder Succinat sowie Anionen von Polycarbonsäuren mit bis zu 3000 COOH-Gruppen.

Einzelheiten der Herstellung können den Beispielen entnommen werden.

Die erfindungsgemäßen Verbindungen eignen sich zum Stabilisieren von organischem Material, speziell von Kunststoffen, gegen den Abbau durch Licht und Wärme. Sie werden den zu stabilisierenden Kunststoffen in einer Konzentration von 0,01 bis 5 Gew.%, vorzugsweise 0,02 bis 1 Gew.% vor, während oder nach der Polymerbildung zugesetzt.

Zur Vermischung der erfindungsgemäßen Verbindungen mit den zu stabilisierenden Kunststoffen können alle bekannten Vorrichtungen und Methoden zum Einmischen von Stabilisierungsmitteln oder anderen Zusätzen in Polymere angewandt werden.

Die durch eine der erfindungsgemäßen Verbindungen stabilisierten Kunststoffe können gegebenenfalls noch weitere Additive enthalten, beispielsweise Antioxidantien, Lichtstabilisierungsmittel, Metalldesaktivatoren, antistatische Mittel, flammhemmende Mittel, Pigmente und Füllstoffe.

Antioxidantien und Lichtstabilisatoren, die den Kunststoffen neben den erfindungsgemäßen Verbindungen zugesetzt werden können, sind z. B. Verbindungen auf der Basis sterisch gehinderter Phenole oder Schwefel oder Phosphor enthaltende Costabilisatoren.

Als derartige phenolische Antioxidationsmittel seien beispielsweise 2,6-Di-tert.-butyl-4-methylphenol, n-Octadecyl-β-(3,5-di-tert.-butyl-4-hydroxyphenyl)-propionat, 1,1,3-Tris-(2-methyl-4-hydroxy-5-tert.-butylphenyl)-butan, 1,3,5-Trimethyl-2,4,6-tris-(3,5-di-tert.-butyl-4-hydroxybenzyl)-benzol, 1,3,5-Tris-(3,5-di-tert.-butyl-4-hydroxybenzyl)-isocyanurat, 1,3,5-Tris-[β-(3,5-di-tert.-butyl-4-hydroxyphenyl)-propionyloxyethyl]-isocyanurat, 1,3,5-Tris-(2,6-di-methyl-3-hydroxy-4-tert.-butylbenzyl)-isocyanurat, Pentaerythrit-tetrakis-[β-(3,5-di-tert.-butyl-4-hydroxyphenyl)-propionat] etc. erwähnt.

Als phosphorhaltige Antioxidantien seien beispielsweise Tris-(nonylphenyl)-phosphit, Distearylpentaerythritdiphosphit, Tris-(2,4-di-tert.-butylphenyl)-phosphit, Tris-(2-tert.-butyl-4-methylphenyl)-phosphit, Bis-(2,4-di-tert.-butylphenyl)-pentaerythritdiphosphit, Tetrakis-(2,4-di-tert.-butylphenyl)-4,4′-biphenylendiphosphit etc. erwähnt.

Als Schwefel enthaltende Antioxidationsmittel seien beispielsweise Dilaurylthiodipropionat, Dimyristylthiodipropionat, Distearylthiodipropionat, Pentaerythrittetrakis-(β-laurylthiopropionat), Pentaerythrittetrakis-(β-hexylthiopropionat) etc. erwähnt.

Weitere Antioxidantien und Lichtstabilisatoren, die zusammen mit den erfindungsgemäßen Verbindungen verwendet werden können, sind z. B. 2-(2'-Hydroxyphenyl)-benztriazole, 2-Hydroxybenzophenone, Arylester von Hydroxybenzoesäuren, α-Cyanozimtsäurederivate, Nickelverbindungen oder Oxalsäuredianilide.

Als organische Polymere, die durch die erfindungsgemäßen Verbindungen stabilisiert werden können, seien beispielsweise genannt :

Polymere von Mono- und Diolefinen, wie z. B. Polyethylen niedriger oder hoher Dichte, lineares Polyethylen niedriger Dichte, Polypropylen, Polyisobutylen, Polybuten-1, Polymethylpenten-1, Polyisopren, Polybutadien sowie Copolymerisate von Mono- oder Diolefinen oder Mischungen der genannten Polymeren ;

Copolymerisate von Mono- oder Diolefinen mit anderen Vinylmonomeren wie z. B. Ethylen-Alkylacrylat-Copolymere, Ethylen-Alkylmethacrylat-Copolymere, Ethylen-Vinylacetat-Copolymere oder Ethylen-Acrylsäure-Copolymere ;

Polystyrol ;

Copolymere von Styrol oder α-Methylstyrol mit Dienen oder Acrylderivaten, wie z. B. Styrol-Butadien, Styrol-Acrylnitril, Styrol-Ethylmethacrylat, Styrol-Butadien-Ethylacrylat, Styrol-Acrylnitril-Methylacrylat ;

ABS-, MBS- oder ähnliche Polymere ;

Halogenhaltige Polymere, wie z. B. Polyvinylchlorid, Polyvinylfluorid, Polyvinylidenfluorid sowie deren Copolymere ;

Polymere die sich von α,β-ungesättigten Säuren und deren Derivaten ableiten, wie Polyacrylate und Polymethacrylate, Polyacrylamide und Polyacrylnitrile ;

Polymere, die sich von ungesätttigten Alkoholen und Aminen bzw. deren Acylderivaten oder Acetalen ableiten, wie Polyvinylakohol oder Polyvinylacetat ;

Polyurethane, Polyamide, Polyharnstoffe, Polyester, Polycarbonate, Polysulfone und Polyethersulfone.

Weitere organische Polymere, die mit den erfindungsgemäßen Verbindungen stabilisiert werden können, stellen Industrielackierungen dar. Unter diesen sind Einbrennlackierungen, unter diesen wiederum Fahrzeuglackierungen, vorzugsweise Zweischichtlackierungen, besonders hervorzuheben.

Auch hier können zusätzlich die bereits aufgeführten Antioxidantien und Lichtschutzmittel verwendet werden.

Die erfindungsgemäßen Verbindungen können, soweit sie fest sind, in fester oder gelöster Form, oder, wenn sie flüssig sind, in Substanz dem Lack zugesetzt werden. Ihre gute Löslichkeit in Lacksystemen ist dabei von besonderem Vorteil.

Bevorzugt ist die Verwendung der erfindungsgemäßen Verbindungen in Polyolefinen, vorzugsweise Ethylen- und Propylenpolymerisaten.

### Beispiel 1

2,5-Dimethylfuran-3-carbonsäure-2,2,6,6-tetramethyl-4-piperidinylester

154 g (1 Mol) 2,5-Dimethylfuran-3-carbonsäuremethylester und 157 g (1 Mol) 4-Hydroxy-2,2,6,6-tetramethylpiperidin werden zusammen mit 10 g Tetrabutylorthotitanat 10 Stunden auf ca. 190 °C erhitzt, wobei das entstehende Methanol abdestilliert wird. Nach der Umsetzung wird abgekühlt und die Reaktionsmasse in Essigester gelöst. Die Lösung wird zunächst mit 10 %iger Sodalösung, dann mit Wasser ausgeschüttelt, getrocknet und eingeengt. Der Rückstand wird im Vakuum destilliert. Das gewünschte Produkt destilliert bei 142 °C/266,6 Pa (2 mmHg) als fast farbloses Öl, welches bald zu einem Feststoff vom Schmp. 59 bis 61 °C erstarrt.

### Beispiel 2

2,5-Dimethylfuran-3-carbonsäure-1,2,2,6,6-pentamethyl-4-piperidinylester

Verwendet man in Beispiel 1 4-Hydroxy-1,2,2,6,6-pentamethylpiperidin anstelle von 4-Hydroxy-2,2,6,6-tetramethylpiperidin, so erhält man das Produkt als fast farbloses Öl vom Sdp. 120 bis 122 °C/20 Pa (0,15 mmHg).

### Beispiel 3

2,4-Dimethylfuran-3-carbonsäure-2,2,6,6-tetramethyl-4-piperidinylester

Verwendet man in Beispiel 1 2,4-Dimethylfuran-3-carbonsäuremethylester anstelle von 2,5-Dimethylfuran-3-carbonsäuremethylester, so erhält man das Produkt als fast farbloses Öl vom Sdp. 136 bis 138 °C/133,3 Pa (1 mmHg).

Beispiel 4

2,4,5-Trimethylfuran-3-carbonsäure-2,2,6,6-tetramethyl-4-piperidinylester

Verwendet man in Beispiel 1 2,4,5-Trimethylfuran-3-carbonsäuremethylester anstelle von 2,5-Dimethylfuran-3-carbonsäuremethylester, so erhält man das Produkt als fast farbloses Öl vom Sdp. 137 bis 139 °C/66,7 Pa (0,5 mmHg) welches alsbald zu einem Feststoff vom Schmp. 54 bis 58 °C erstarrt.

Beispiel 5

2,4,5-Trimethylfuran-3-carbonsäure-1,2,2,6,6-pentamethyl-4-piperidinylester

Setzt man 2,4,5-Trimethylfuran-3-carbonsäuremethylester und 4-Hydroxy-1,2,2,6,6-pentamethylpiperidin nach der in Beispiel 1 angegebenen Vorschrift um, so erhält man das Produkt als fast farbloses Öl vom Sdp. 138 bis 140 °C/53,3 Pa (0,4 mmHg), welches zu einem Feststoff vom Schmp. 55 bis 56 °C erstarrt.

Beispiel 6

2,5-Dimethylfuran-3-carbonsäure-2,2,6,6-tetramethyl-4-piperidinylamid

156 g (1 Mol) 4-Amino-2,2,6,6-tetramethylpiperidin und 79 g (0,5 Mol) 2,5-Dimethylfuran-3-carbonsäurechlorid werden in 1 l Toluol 24 Stunden rückflußgekocht. Dann wird vom Niederschlag abfiltriert, die Toluollösung mit $NaHCO_3$-Lösung und Wasser ausgeschüttelt, getrocknet und eingeengt. Der Rückstand weist nach Umkristallisieren aus Cyclohexan einen Schmelzpunkt von 116 bis 118 °C auf.

Beispiel 7

2,5-Dimethylfuran-3-carbonsäure-2,2,6,6-tetramethyl-N-(2-hydroxyethyl)-4-piperidinylester

50 g des in Beispiel 1 beschriebenen Produkts werden in einem Autoklaven in 50 %igem wäßrigen Ethanol mit Ethylenoxid bei 110 bis 120 °C und einem Anfangsdruck von 6 bar umgesetzt und die Umsetzung beendet, wenn der Druck auf 1 bar abgesunken ist.

Das dabei erhaltene Produkt wird durch Ausrühen mit Wasser gereinigt. Es liegt als praktisch weißer Feststoff mit einem Schmelzpunkt von 83 bis 85 °C vor.

Beispiel 8

2,5-Dimethylfuran-3-carbonsäure-N-butyl-N-(2,2,6,6-tetramethyl-4-piperidinyl)amid

158,5 g (1 Mol) 2,5-Dimethylfuran-3-carbonsäurechlorid und 212 g (2 Mol) 4-N-Butylamino-2,2,6,6-tetramethylpiperidin werden in 1,5 l Toluol zusammen mit 55 g wasserfreier Soda 24 Stunden rückflußgekocht. Man läßt abkühlen und saugt den Niederschlag ab. Der Niederschlag wird mit 1 l Wasser aufgerührt und zweimal mit 0,5 l Essigester extrahiert. Die vereinigten Essigesterphasen werden getrocknet und im Wasserstrahlvakuum eingeengt. Der kristallisierte Rückstand wird mit wenig Petrolether aufgeschlämmt, abgesaugt und getrocknet. Er weist einen Schmelzpunkt von 78-79 °C auf und ist gaschromatographisch rein.

Beispiel 9

2,5-Dimethylfuran-3-carbonsäure-N-butyl-N-(1,2,2,6,6-pentamethyl-4-piperidinyl)amid

100,2 g (0,3 Mol) 2,5-Dimethylfuran-3-carbonsäure-N-butyl-N-(2,2,6,6-tetramethyl-4-piperidinyl)amid (Herstellbeispiel 8) werden mit 100 g 37 %iger wäßriger Formaldehydlösung und 30 g Ameisensäure 7 Stunden unter Rückfluß gekocht.

Nach dem Abkühlen wird 1 l Wasser zugesetzt und mit 5 %iger wäßriger Kalilauge alkalisch gestellt. Dann wird zweimal mit 0,5 l Essigester extrahiert. Die vereinigten Essigesterphasen werden über $MgSO_4$ getrocknet und im Wasserstrahlvolumen eingeengt. Das Produkt bleibt als blaßgelbes Öl zurück.

Beispiel 10

Bis-(N-1,6-hexamethylen-N-2,2,6,6-tetramethyl-4-piperidinyl)-2,5-dimethylfuran-3-carbonsäureamid

158,5 g (1 Mol) 2,5-Dimethylfuran-3-carbonsäurechlorid und 197 g (0,5 Mol) Bis-4-(1,6-hexamethylen)-amino-2,2,6,6-tetramethylpiperidin werden in 1,5 l Toluol zusammen mit 55 g wasserfreier Soda 24

Stunden unter Rückfluß gekocht. Dann läßt man abkühlen, saugt den Niederschlag ab und trocknet ihn bei 80 °C im Vakuum. Anschließend wird der Niederschlag in 2 l Wasser eingerührt und die Suspension mit verdünnter Natronlauge alkalisch gemacht. Der Niederschlag wird abgesaugt, mit wenig Wasser neutralgewaschen und bei 80 °C im Vakuum getrocknet. Das Produkt weist nach dem Umkristallisieren aus Cyclohexan einen Schmelzpunkt von 162-4 °C auf.

Beispiel 11

Salz von Bis-(N-1,6-hexamethylen-N-2,2,6,6-tetramethyl-4-piperidinyl)-2,5-dimethylfuran-3-carbonsäureamid mit Polyacrylsäure mit ca. 70 Einheiten

64 g (0,1 Mol) des Carbonsäureamids aus Herstellbeispiel 10 und 7,2 g gepulverte, sorgfältig entwässerte Polyacrylsäure mit einem Molekulargewicht von ca. 5 000 werden in Methanol unter leichtem Erwärmen gerührt, bis eine klare Lösung entstanden ist. Die Lösung wird im Wasserstrahlvakuum bis zur Trockene eingeengt und der Rückstand pulverisiert. Er weist einen Schmelzpunkt von 156-8 °C auf.

Weitere erfindungsgemäße Verbindungen sind in den Tabellen 1 bis 5 aufgeführt:

Die Bezeichnungen beziehen sich auf Formel I.

Tabelle 1 $R^1 = R^2 = CH_3$; $R^3 = H$

| Beispiel | $R^4$ | Smp. bzw. Sdp. |
|---|---|---|
| 12 | wie Bsp. 1, Salz mit 2,4-Dimethylglutarsäure | 156-8 °C |
| 13 | wie Bsp. 1, Salz mit Adipinsäure | 176-8 °C |
| 14 | wie Bsp. 1, Salz mit Polyacrylsäure (ca.28-42 Einheiten) | 115-20 °C |
| 15 | wie Bsp. 1, Salz mit Polyacrylsäure (ca. 70 Einheiten) | 148-150 °C |
| 16 | wie Bsp. 1, Salz mit Polyacrylsäure (ca. 2100-2800 Einheiten) | ca. 300 °C |
| 17 | wie Bsp. 6, Salz mit 2,4-Dimethylglutarsäure | 180-2 °C |
| 18 | wie Bsp. 6, Salz mit Polyacrylsäure (ca. 28-42 Einheiten) | 122-6 °C |
| 19 | wie Bsp. 6, Salz mit Polyacrylsäure (ca. 70 Einheiten) | 156-8 °C |
| 20 | 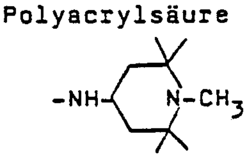 | 126-8 °C |
| 21 | wie Bsp. 8, Salz mit 2,4-Dimethylglutarsäure | 143-5 °C |

Tabelle 1 (Fortsetzung)

| Beispiel | $R^4$ | Smp. bzw. Sdp. |
|---|---|---|
| 22 | wie Bsp. 8, Salz mit Adipinsäure | |
| 23 | wie Bsp. 8, Salz mit Polyacrylsäure (ca. 28-42 Einheiten) | 78-82 °C |
| 24 | wie Bsp. 8, Salz mit Polyacrylsäure (ca. 70 Einheiten) | 80-2 °C |
| 25 | 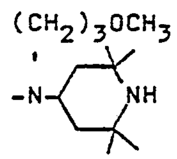 | 50 °C |
| 26 | 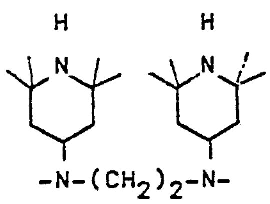 | 192-4 °C |
| 27 | 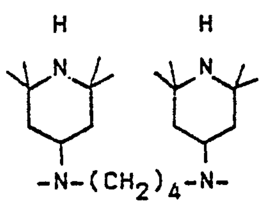 | 115-20 °C |
| 28 | 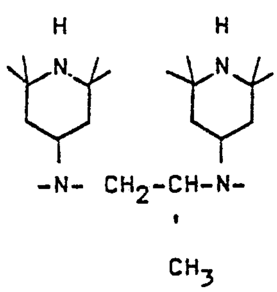 | |
| 29 | 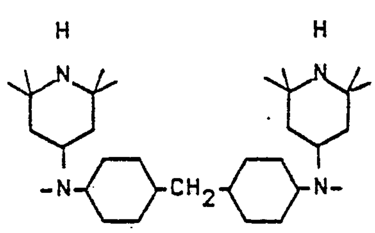 | 72-6 °C |

Tabelle 1 (Fortsetzung)

| Beispiel | $R^4$ | Smp. bzw. Sdp. |
|---|---|---|
| 30 | 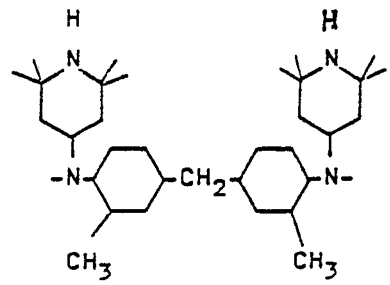 | |
| 31 | 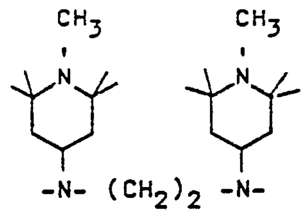 | |
| 32 | 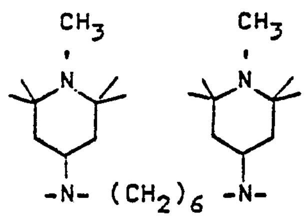 | |
| 33 | 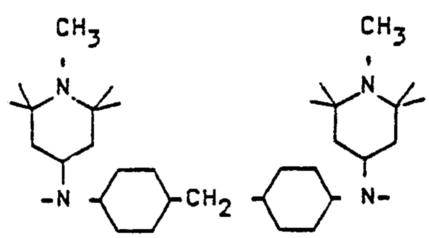 | |
| 34 | 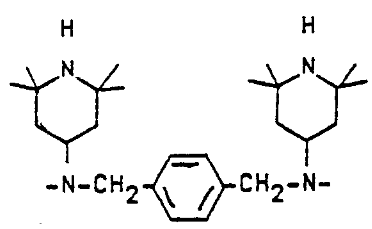 | |
| 35 | 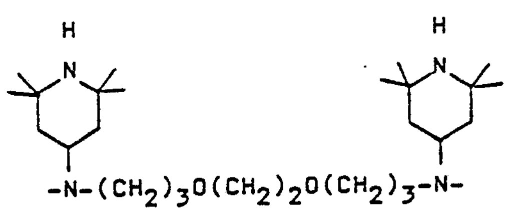 | |

Tabelle 2 $R^1 = R^3 = CH_3$ ; $R^2 = H$

| Beispiel | $R^4$ | Smp. bzw. Sdp. |
|---|---|---|
| 36 | wie Bsp. 3, Salz mit Adipinsäure | 182-4 °C |
| 37 | wie Bsp. 3, Salz mit Polyacrylsäure (ca. 70 Einheiten) | 132-5 °C |
| 38 | 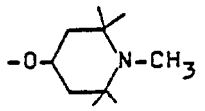 | 120 °C/40 Pa (0.3 mm Hg) |
| 39 | 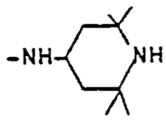 | 170-3 °C |
| 40 | wie Bsp. 39, Salz mit Adipinsäure | 178-80 °C |
| 41 | 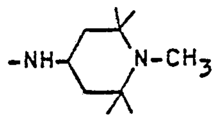 | |
| 42 | 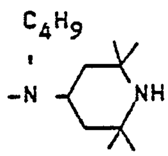 | 100-2 °C |
| 43 | wie Bsp. 42, Salz mit Adipinsäure | |
| 44 | 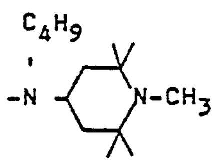 | |
| 45 | 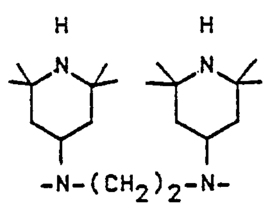 | |
| 46 | 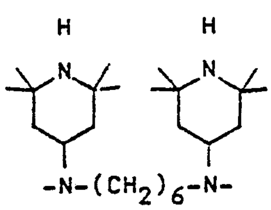 | 172-4 °C |
| 47 | 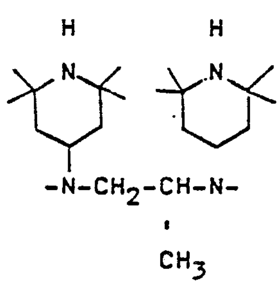 | |

Tabelle 2 (Fortsetzung)

| Beispiel | $R^4$ | Smp. bzw. Sdp. |
|---|---|---|
| 48 | 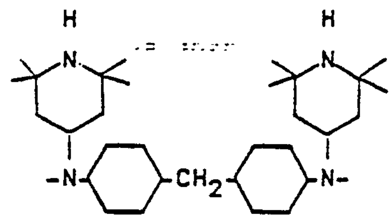 | 128-30 °C |
| 49 | 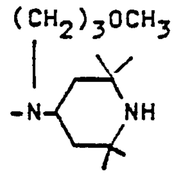 | 78-79 °C |

Tabelle 3 $R^1 = R^2 = R^3 = CH_3$

| Beispiel | $R^4$ | Smp. bzw. Sdp. |
|---|---|---|
| 50 | wie Bsp. 4, Salz mit Adipinsäure | 170-2 °C |
| 51 | wie Bsp. 4, Salz mit Polyacrylsäure (ca. 70 Einheiten) | 142-6 °C |
| 52 | 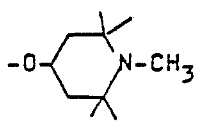 | 55-6 °C<br>138-40 °C/53,3 Pa<br>(0.4 mm Hg) |
| 53 | 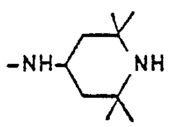 | 150-5 °C |
| 54 | 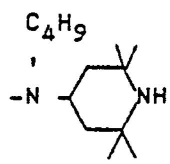 | |
| 55 | 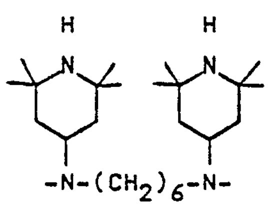 | |
| 56 | 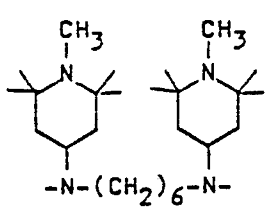 | |

Tabelle 4 $R^1 = CH_3$ ; $R^2 = R^3 = H$

| Beispiel | $R^4$ | Smp. bzw. Sdp. |
|---|---|---|
| 57 | 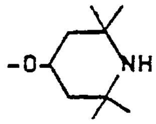 | viskos |
| 58 | wie Bsp. 57, Salz mit Polyacrylsäure (ca. 70 Einheiten) | 135-40 °C |
| 59 | 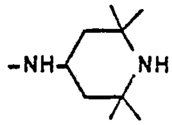 | |
| 60 | 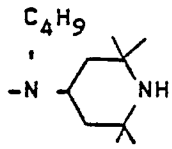 | |
| 61 | 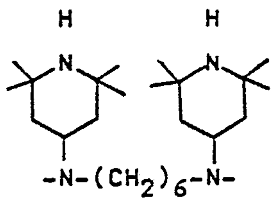 | |

Tabelle 5 Derivate verschiedener Furancarbonsäuren

| Beispiel | $R^1$ | $R^2$ | $R^3$ | $R^4$ | Smp. bzw. Sdp. |
|---|---|---|---|---|---|
| 62 | $CH_3$ | H | $C_2H_5$ | 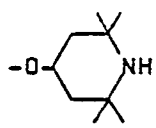 | 50-2 °C |
| 63 | $CH_3$ | H | $C_2H_5$ | 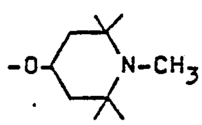 | 180-6° C/66,7 Pa (0.5 mm Hg) |
| 64 | $CH_3$ | H | $C_2H_5$ | 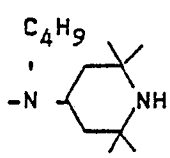 | |
| 65 | $CH_3$ | $C_2H_5$ | H | 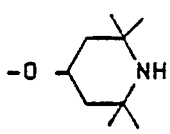 | |
| 66 | $CH_3$ | $C_2H_5$ | H | 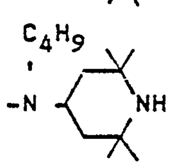 | |
| 67 | $CH_3$ | $C_6H_5$ | H | 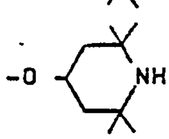 | 103-5 °C |

Anwendungsbeispiele

1) Stabilisierung von Polyethylen mit der Verbindung aus Beispiel 1

a) 0,25 Teile der Verbindung aus Beispiel 1 werden in 100 Teilen Polyethylen niedriger Dichte (1 840 D der Fa. BASF) durch zweimaliges Extrudieren bei 180 °C eingearbeitet und zu 200 µm dicken Platten gepreßt. Nach 14-tägiger Lagerung im Dunkeln bei 25 °C zeigt die Oberfläche der Platten keinen Belag.

b) Die nach a) hergestellten Platten werden in einem QUV-Schnellbewitterungs-Prüfgerät auf ihre Bewitterungsstabilität getestet. Die Alterung wird durch Messen der « CO-Zahl » nach bestimmten Zeitintervallen bestimmt. Der Beginn der Versprödung gilt als erreicht, wenn die CO-Zahl einen Wert von 10 erreicht hat. Die Testergebnisse sind in Tabelle 1 zusammengefaßt. Als Vergleich wird der Furan-2-carbonsäure-2,2,6,6-tetramethyl-4-piperidinylester unter den gleichen Bedingungen getestet.

Analog Beispiel 1a) wurden Prüfkörper mit den Verbindungen aus den Beispielen 2, 3 und 8 angefertigt und analog Beispiel 1b) getestet. Die Ergebnisse sind in Tabelle 1 zusammengefaßt.

Tabelle 1

| | Belichtungszeit in (h) | | | | | | | | |
|---|---|---|---|---|---|---|---|---|---|
| Verbindung | 500 | 1000 | 1500 | 2000 | 2500 | 3000 | 3500 | 4000 | 4500 |
| Beispiel 1 | 0,33 | 0,5 | 0,77 | 1,55 | 7,4 | 10 | | | |
| Beispiel 2 | 0,30 | 0,51 | 0,68 | 1,1 | 3,5 | 10 | | | |
| Beispiel 3 | | 0,18 | 0,24 | 0,48 | 0,90 | 1,83 | 2,56 | 3,39 | 7,08 |
| Beispiel 8 | | 0,24 | 0,41 | 1,03 | 2,01 | 3,75 | 7,74 | | |
| Beispiel 21 | | 0,07 | 0,13 | 0,17 | 0,25 | | | | |
| Beispiel 67 | | 0,22 | 0,32 | 0,49 | 0,94 | | | | |
| Furan-2-carbonsäure-2,2,6,6-tetramethyl-4--piperidinylester | 0,5 | 1,55 | 6,07 | | | | | | |
| ohne Stabilisator | 5 | | | | | | | | |

2) Stabilisierung von Polypropylen

a) 0,25 Teile der Verbindung des entsprechenden Beispiels werden in 100 Teilen Polypropylen (1 320 H der Fa. BASF) durch zweimaliges Extrudieren bei 220 °C eingearbeitet und zu 200 µm dicken Platten gepreßt. Nach 14-tägiger Lagerung im Dunkeln bei 25 °C zeigt die Oberfläche der Platten keinen Belag.

b) Die nach a) hergestellten Platten werden in einem QUV-Schnellbewitterungs-Prüfgerät auf ihre Bewitterungsstabilität getestet. Die Alterung wird durch Messen der « CO-Zahl » nach bestimmten Zeitintervallen bestimmt. Der Beginn der Versprödung wird mechanisch ermittelt. Die Testergebnisse sind in Tabelle 2 zusammengefaßt.

Tabelle 2

| Verbindung | Zeit bis zur Versprödung in (h) |
|---|---|
| Beispiel 6 | 1 730 |
| Beispiel 8 | 1 920 |
| Beispiel 10 | 1 740 |
| Beispiel 11 | 1 770 |
| Beispiel 21 | 1 700 |
| Beispiel 25 | 1 720 |
| Beispiel 26 | 2 000 |
| Beispiel 27 | 1 770 |
| Beispiel 29 | 1 620 |
| Beispiel 39 | 1 920 |
| Beispiel 53 | 1 740 |
| Furan-2-carbonsäure-2,2,6,6-tetramethyl-4-piperidinylester | 950 |

## Patentansprüche

1. Furancarbonsäurederivate der allgemeinen Formel

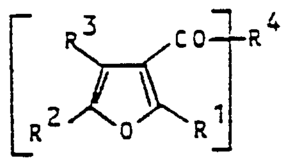

(I)

in der

$R^1$ bis $R^3$ unabhängig voneinander Wasserstoff, $C_1$-$C_8$Alkyl, Cyclohexyl oder Phenyl, jedoch nicht mehr als zwei dieser Reste gleichzeitig Wasserstoff und

$R^4$ ein Rest der Formel

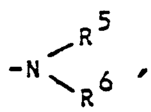

,

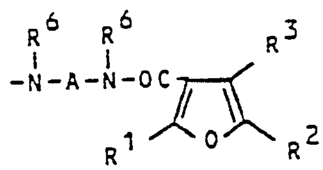

oder $OR^6$ sind, wobei

A lineares oder verzweigtes, gegebenenfalls ein oder mehrere Ethersauerstoff enthaltendes $C_2$- bis $C_{12}$-Alkylen,

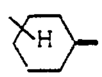

,

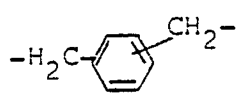

,

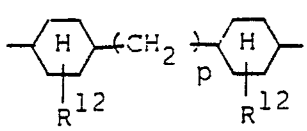

oder

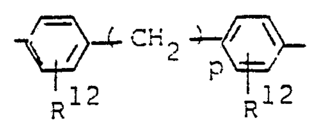

worin $R^{12}$ Wasserstoff oder Methyl und p 0, 1 oder 2 bedeuten,

$R^5$ Wasserstoff, $C_2$-bis $C_6$-Alkenyl gegebenenfalls bis zu 3 mal durch Sauerstoff unterbrochenes $C_1$- bis $C_{12}$-Alkyl oder $C_5$- bis $C_7$-Cycloalkyl und

$R^6$ ein Rest der Formel

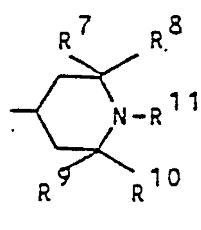

sind, in der

$R^7$ bis $R^{10}$ $C_1$- bis $C_4$-Alkyl oder

$R^7$ und $R^8$ und $R^9$ und $R^{10}$ zusammen eine Tetra- oder Pentamethylenbrücke und

$R^{11}$ Wasserstoff, $C_1$- bis $C_8$-Alkyl, $C_3$- bis $C_8$-Alkenyl, $C_2$- bis $C_4$-Hydroxyalkyl oder Aralkyl bedeuten sowie deren Salze.

2. Verbindungen gemäß Anspruch 1, wobei A eine lineare oder verzweigte, gegebenenfalls ein oder mehrere Ethersauerstoff enthaltende $C_2$- bis $C_{12}$-Alkylengruppe, ein Aralkylenrest der Formel

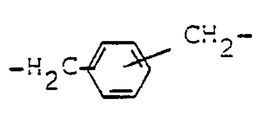

oder

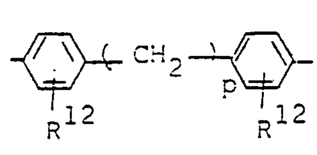

in der $R^2$ Wasserstoff oder Methyl und p 0, 1 oder 2 bedeuten, oder ein Cycloalkylgruppen enthaltender $C_6$- bis $C_{16}$-Rest ist.

3. Verbindungen gemäß Anspruch 1, wobei $R^7$ bis $R^{10}$ Methylgruppen sind.

4. Verbindungen gemäß Anspruch 1, wobei $R^{11}$ Wasserstoff oder Methyl ist.

5. Piperidinylester und -amide gemäß Anspruch 1 der 2,4- oder 2,5-Dimethylfuran-3-carbonsäure oder der 2,4,5-Trimethylfuran-3-carbonsäure.

6. Verwendung der Verbindungen gemäß Anspruch 1 zum Stabilisieren von organischem Material.

7. Verwendung gemäß Anspruch 6 zum Stabilisieren von Polyolefinen.

8. Stabilisiertes organisches Material, enthaltend eine Verbindung gemäß Anspruch 1.

## Claims

1. A furancarboxylic acid derivative of the formula

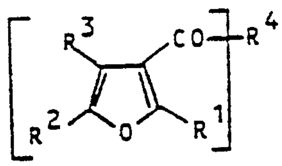

(I)

where

$R^1$, $R^2$, and $R^3$ independently of one another are each hydrogen, $C_1$-$C_8$-alkyl, cyclohexyl or phenyl, but not more than two of these radicals are simultaneously hydrogen, and

$R^4$ is a radical of the formula

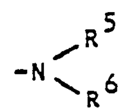 , 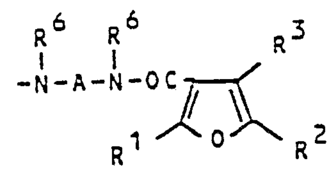

or $OR^6$, where

A is linear or branched $C_2$-$C_{12}$-alkylene which may contain one or more ether oxygen atoms

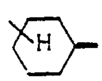 , 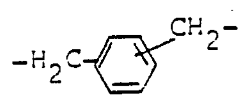 ,

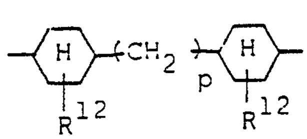 or 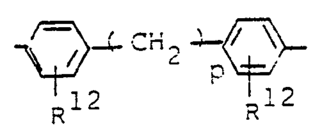

where $R^{12}$ is hydrogen or methyl and p is 0, 1 or 2,

$R^5$ is hydrogen, $C_2$-$C_6$-alkenyl, $C_5$-$C_7$-cycloalkyl or $C_1$-$C_{12}$-alkyl which may or may not be interrupted by 1, 2 or 3 oxygen atoms, and

$R^6$ is a radical of the formula

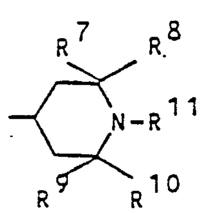

where

$R^7$, $R^8$, $R^9$ and $R^{10}$ are each $C_1$-$C_4$-alkyl, or

$R^7$ and $R^8$ and $R^9$ and $R^{10}$ together form a tetramethylene or pentamethylene bridge, and $R^{11}$ is hydrogen, $C_1$-$C_8$-alkyl, $C_3$-$C_8$-alkenyl, $C_2$-$C_4$-hydroxyalkyl or aralkyl, and its salts.

2. A compound as claimed in claim 1, wherein A is a straight-chain or branched $C_2$-$C_{12}$-alkylene group which may or may not contain one or more ether oxygen atoms, of the formula

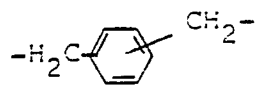
or
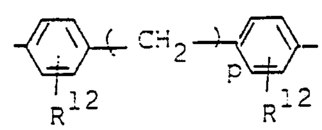

where $R^2$ is hydrogen or methyl, and p is 0, 1 or 2, or a cycloalkyl-containing $C_6$-$C_{16}$ radical.

3. A compound as claimed in claim 1, wherein $R^7$, $R^8$, $R^9$ and $R^{10}$ are each methyl.

4. A compound as claimed in claim 1, wherein $R^{11}$ is hydrogen or methyl.

5. A piperidinyl ester or amide as claimed in claim 1, which is based on 2,4- or 2,5-dimethylfuran-3-carboxylic acid or on 2,4,5-trimethylfuran-3-carboxylic acid.

6. Use of a compound as claimed in claim 1 for stabilizing organic material.

7. Use as claimed in claim 6 for stabilizing polyolefins.

8. A stabilized organic material, containing a compound as claimed in claim 1.

**Revendications**

1. Dérivés d'acide furanne-carboxylique de formule générale

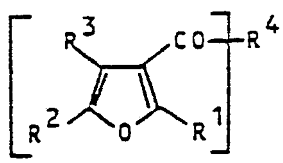
(I)

dans laquelle

$R^1$ à $R^3$ indépendamment l'un de l'autre sont des atomes d'hydrogène ou des radicaux alkyle en $C_1$-$C_8$, cyclohexyle ou phényle, mais pas plus de deux de ces radicaux en même temps que l'hydrogène, et

$R^4$ est un radical de formule

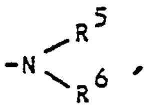
,
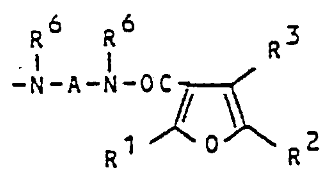

ou $OR^6$, où

A est un groupement alkylène en $C_2$-$C_{12}$ linéaire ou ramifié, contenant éventuellement un ou plusieurs ponts oxygène,

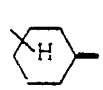
,
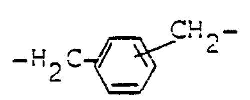
,

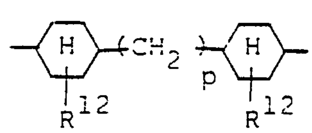
ou
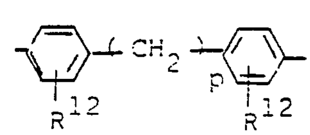

où $R^{12}$ représente un atome d'hydrogène ou un groupement méthyle et p est 0,1 ou 2,

$R^5$ est un atome d'hydrogène ou un groupement alcényle en $C_2$-$C_6$, alkyle en $C_1$-$C_{12}$ éventuellement interrompu jusqu'à 3 fois par de l'oxygène, ou cycloalkyle en $C_5$-$C_7$,

$R^6$ est un radical de formule

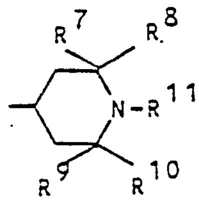

dans laquelle

$R^7$ à $R^{10}$ sont des groupements alkyle en $C_1$-$C_4$,

$R^7$ et $R^8$ ou $R^9$ et $R^{10}$ forment ensemble un pont tétra- ou pentaméthylène,

$R^{11}$ est un atome d'hydrogène ou un groupement alkyle en $C_1$-$C_8$, alcényle en $C_3$-$C_8$, hydroxyalkyle en $C_2$-$C_4$ ou aralkyle, ainsi que leurs sels.

2. Dérivés selon la revendication 1, où A est un groupement alkylène en $C_2$-$C_{12}$ linéaire ou ramifié contenant éventuellement un ou plusieurs ponts oxygène, un groupement aralkylène de formule

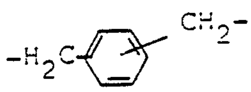

ou

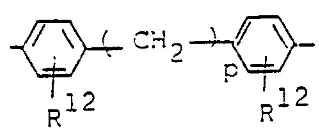

dans laquelle $R^2$ est un atome d'hydrogène ou un radical méthyle et p est 0,1 ou 2, ou un groupement en $C_6$-$C_{16}$ contenant des groupements cycloalkyle.

3. Dérivés selon la revendication 1, où $R^7$ à $R^{10}$ sont des groupements méthyle.

4. Dérivés selon la revendication 1, où $R^{11}$ est un atome d'hydrogène ou un groupement méthyle.

5. Esters et amides pipéridinyliques selon la revendication 1, de l'acide 2,4- ou 2,5-diméthylfuranne-3-carboxylique ou de l'acide 2,4,5-triméthylfuranne-3-carboxylique.

6. Utilisation des dérivés selon la revendication 1, pour stabiliser des matières organiques.

7. Utilisation selon la revendication 6, pour stabiliser des polyoléfines.

8. Matières organiques stabilisées, contenant un dérivé selon la revendication 1.